# EUROPÄISCHE PATENTSCHRIFT

(11) **EP 3 119 458 B1**
(45) Veröffentlichungstag und Bekanntmachung des Hinweises auf die Patenterteilung: **25.07.2018**
(21) Anmeldenummer: 15702454.8
(22) Anmeldetag: 30.01.2015
(51) Int. Cl.: A61M 15/00, A61M 11/02, B05B 11/00, A61M 16/20

(54) **SPENDER MIT EINEM SYSTEM ZUR ERFASSUNG VON AUSTRAGVORGÄNGEN UND VERFAHREN ZUR HERSTELLUNG EINES SOLCHEN SPENDERS**
DISPENSER HAVING A SYSTEM FOR DETECTING DISCHARGE PROCESSES AND PROCESS FOR MANUFACTURING SAID DISPENSER
DISTRIBUTEUR ÉQUIPÉ D'UN SYSTÈME DE DÉTECTION DE PROCESSUS DE DÉLIVRANCE ET PROCÉDÉ DE FABRICATION D'UN TEL DISTRIBUTEUR

(30) Priorität: 17.03.2014 DE 102014204940
(43) Veröffentlichungstag der Anmeldung: 25.01.2017
(73) Patentinhaber: Aptar Radolfzell GmbH, 78315 Radolfzell (DE)
(72) Erfinder: HELMLINGER, Michael, 78315 Radolfzell-Böhringen (DE)
(74) Vertreter: Patentanwaltskanzlei Cartagena
(86) Internationale Anmeldenummer: PCT/EP2015/051988
(87) Internationale Veröffentlichungsnummer: WO 2015/139874

(56) Entgegenhaltungen:
- WO-A1-2004/091806
- WO-A1-2005/080001
- DE-A1-102006 036 962
- DE-A1-102010 048 085
- GB-A- 2 451 833
- US-A1- 2010 192 948

## Beschreibung

### ANWENDUNGSGEBIET UND STAND DER TECHNIK

Die Erfindung betrifft einen Spender zum Austrag pharmazeutischer Medien mit einem Flüssigkeitsspeicher, einem Basiskörper, einer gegenüber dem Basiskörper verlagerbaren Betätigungshandhabe zur Verursachung eines Austragvorgangs von Medium aus dem Flüssigkeitsspeicher in einer Umgebung und einem elektronischen Erfassungssystem zur Erfassung von Austragvorgängen.

Gattungsgemäße Spender sind aus dem Stand der Technik allgemein bekannt. So schlägt beispielsweise die DE 10 2006 036 962 A1 einen Spender vor, der über ein Zählmodul verfügt, welches dem Benutzer Auskunft über bereits erfolgte Austragvorgänge gibt. Gezählt wird durch dieses Zählmodul das Auftreten von Relativverlagerungen von Bauteilen des Spenders, die im Zuge eines Austragvorgangs manuell gegeneinander verlagert werden.

Eine Problematik, die sich bei gattungsgemäßen Spendern in der Vergangenheit gezeigt hat, liegt darin, dass ein exaktes Abstimmen des Erfassungssystems in Hinblick auf den Austragvorgang schwer möglich ist. Dies bedeutet, dass die Gefahr besteht, dass ein vermeintlicher Austragvorgang erfasst wird, der tatsächlich jedoch nicht stattgefunden hat, da die Verlagerung der Betätigungshandhabe nicht ausreichend weit erfolgt ist. Das Problem kann sich auch darin zeigen, dass ein Austragvorgang stattgefunden hat, dieser jedoch nicht erfasst wurde, da eine hierfür ausreichend weite Verlagerung der Betätigungshandhabe nicht stattgefunden hat.

Es hat sich gezeigt, dass dieses Problem vor allen Dingen deshalb gegeben ist, da Bauteiltoleranzen am Spender zu geringfügig voneinander abweichenden Betätigungsstrecken der Betätigungshandhabe bis zum Einsetzen eines Austragvorgangs führen. Es kann somit geschehen, dass zwei bestimmungsgemäß baugleiche Spender sich derart hinsichtlich ihres Verhaltens in Reaktion auf eine Betätigung der Betätigungshandhabe unterscheiden, dass beim gleichen Verlagerungsweg im Falle eines der Spender ein Austragvorgang stattfindet, während beim anderen Spender noch kein Austragvorgang stattfindet.

Aus dem Stand der Technik sind verschiedene Spender bekannt, die für unterschiedliche Funktionen über einen Speicher verfügen. Hierzu zählen die Dokumente WO 2005/080001 A1, US 2010/0192948 A1 und GB 2451833 A. Aus dem Dokument DE 102006036962 A1 ist ein Spender mit einem elektronischen Zählwerk bekannt. Aus dem WO 2004/091806 A1 ist Prüfeinrichtung für Spender bekannt.

### AUFGABE UND LÖSUNG

Aufgabe der Erfindung ist es, einen Spender zu schaffen, der die insbesondere durch Bauteiltoleranzen bedingte oben geschilderte Problemstellung überwindet.

Erfindungsgemäß wird dies dadurch erreicht, dass das Erfassungssystem einen Sensor aufweist, der in Abhängigkeit der Verlagerung eines Referenzbauteils, welches mit der Betätigungshandhabe mechanisch gekoppelt ist, ein Ausgangssignal erzeugt, wobei der Sensor zur Erzeugung von Ausgangssignalen mindestens zwei unterscheidbare Arten in Abhängigkeit des Maßes der Verlagerung ausgebildet ist. Weiterhin weist das Erfassungssystem eines erfindungsgemäßen Spenders einen elektronischen Kalibrierungsspeicher auf, in welchem Kalibrierungsdaten zum Austragverhalten des Spenders abgelegt sind.

Bei einem erfindungsgemäßen Spender ist somit eine Betätigungshandhabe vorgesehen, mittels derer auf manuelle Weise ein Austragvorgang hervorgerufen werden kann. Diese Wirkkopplung kann beispielsweise dadurch geschaffen sein, dass mittels der Betätigungshandhabe eine Kolbenpumpe betätigt wird oder ein Auslassventil geöffnet wird. Dabei ist die Betätigungshandhabe mit jenen Komponenten des Spenders, die den Austragvorgang bewirken, derart gekoppelt, dass unter der Annahme vollständig baugleicher Spender der Austragvorgang in einer genau definierten und für alle baugleichen Spender identischen Relativstellung der Betätigungshandhabe gegenüber dem Basiskörper erfolgt.

Die erfindungsgemäßen Maßnahmen helfen dabei, eine Verarbeitung der Austragvorgänge mit geringer Fehlerquote zu erreichen, obwohl zwischen verschiedenen typgleichen Spendern die Relativlage der Betätigungshandhabe gegenüber dem Basiskörper, ab der der Austragvorgang beginnt, voneinander abweicht. Der erfindungsgemäß vorgesehene Sensor, der unmittelbar die Relativbewegung zwischen der Betätigungshandhabe und dem Basiskörper oder aber die Relativbewegung zwischen einem mit der Betätigungshandhabe zwangsgekoppelten anderweitigen Referenzbauteil und dem Basiskörper erfasst, ist in der Lage, in Abhängigkeit des Maßes der Verlagerung mindestens zwei unterschiedliche Arten von Signalen abzugeben. Ein Signal der ersten Art wird dabei bei einem ersten Verlagerungsmaß abgegeben. Ein Signal der zweiten Art wird bei einem zweiten Verlagerungsmaß abgegeben. Ausgehend von einem unbetätigten Zustand des Spenders führt eine manuell verursachte Verlagerung der Betätigungshandhabe somit zunächst zu einem Sensorsignal einer ersten Art und mit fortschreitender Verlagerung anschließend zu einem Sensorsignal einer zweiten Art. Die Signale unterscheiden sich vorzugsweise in Hinblick auf die elektrische Spannung.

Vorzugsweise liegt die Zahl der unterscheidbaren Arten von Signalen noch deutlich höher als zwei, beispielsweise bei mindestens acht Arten von Signalen. Insbesondere vorzugsweise ist der Sensor zur Abgabe eines analogen sich über die Verlagerungsstrecke der Betätigungshandhabe oder des Referenzbauteils stetig ändernden Signals ausgebildet, so dass gleichsam eine unendliche Zahl von Signalarten gegeben ist, die der Sensor erzeugen kann. Eine solche analoge Ausgestaltung ist beispielsweise gegeben, wenn ein Potentiometer vorgesehen ist, welches als Spannungsteiler wirkt und in Abhängigkeit der Verlagerung des Referenzbauteils gegenüber dem Basiskörper einen veränderlichen Spannungswert zur Verfügung stellt.

Die unterschiedlichen Arten von Signalen, die durch den Sensor abgegeben werden können, gestatten es, bei verschiedenen typgleichen Spendern, die aufgrund von Bauteiltoleranzen und Schwankungen in den Materialeigenschaften in unterschiedlichen Relativstellungen der Betätigungshandhabe den Austragvorgang beginnen lassen, jeweils unterschiedliche Signalarten dahingehend auszuwerten, dass der Austragvorgang begonnen hat.

Erfindungsgemäß ist hierfür weiterhin der elektronische Kalibrierungsspeicher vorgesehen, in welchem Kalibrierungsdaten zum Austragverhalten des konkreten Spenders abgelegt sind. Bei diesen Kalibrierungsdaten handelt es sich um Daten, die sich auf den einzelnen Spender beziehen und die zwischen zwei typgleichen Spendern nicht identisch sein müssen. Diese Kalibrierungsdaten repräsentieren die individuellen Besonderheiten eines Einzelspenders in Hinblick auf sein Verhalten in Reaktion auf eine Verlagerung der Betätigungshandhabe. Das Erfassungssystem weist eine Auswerteeinrichtung zur Verarbeitung der Austragsignale auf, welche den Austragvorgang unter Berücksichtigung der Art des Austragsignals auswertet. Dies bedeutet, dass die Auswerteeinrichtung so ausgestaltet ist, dass sie nicht lediglich das Vorhandensein eines Signals, welches sich durch eine Verlagerung der Betätigungshandhabe beliebigen Maßes ergibt, zur Auswertung heranziehen, sondern auch die Art des Signals, welches sich durch die genannte Ausgestaltung des Sensors in unterschiedlicher Weise ergeben kann.

Die Auswerteeinrichtung des erfindungsgemäßen Spenders ist als Zähleinrichtung ausgebildet, wobei diese Zähleinrichtung einen erfolgten Austragvorgang unter Berücksichtigung der Art des Ausgangssignals des Sensors und unter Berücksichtigung von im Kalibrierungsspeicher abgelegten Daten auswertet.

Diese Zähleinrichtung berücksichtigt somit nicht alleine das Ausgangssignal des Sensors oder die Art dieses Ausgangssignals, sondern darüber hinaus auch im Kalibrierungsspeicher individuell für den Spender abgelegte Werte. Diese Werte geben Auskunft darüber, welches Maß an Verlagerung der Betätigungshandhabe gegenüber dem Basiskörper bei dem konkreten Spender erforderlich ist, um ein Austragvorgang zu initiieren, beziehungsweise welche Art eines Ausgangssignals vom Sensor zu erwarten ist, wenn der Austragvorgang tatsächlich beginnt.

So kann bei stark vereinfachter Darstellung beispielsweise im Speicher abgelegt sein, dass das den Sensor bildende Potentiometer eine Teilspannung zwischen einem Ende der Widerstandsschicht und einem Gleitkontakt des Potentiometers aufweist, die bei 2,5 V liegt, wenn der Austragvorgang beginnt. Wenn die Verlagerung der Betätigungshandhabe gegenüber dem Basiskörper erfolgt, wird die Teilspannung in kurzen Abständen durch das Erfassungssystem abgefragt, das Ergebnis jedoch nicht verarbeitet, sofern die gemessene Teilspannung unter dem genannten Wert von 2,5 V liegt. Erst bei 2,5 V und damit genau im Augenblick des Beginnens des Austragvorgangs wird der im Kalibrierungsspeicher abgelegte Wert erreicht oder überschritten und hierdurch ein Zählvorgang vorgenommen, der beispielsweise in der Erhöhung eines Zählregisters um den Wert 1 liegen könnte.

Der Sensor ist insbesondere vorzugsweise unmittelbar zwischen dem die Betätigungshandhabe bildenden Bauteil und der Basis angeordnet, so dass sein Ausgangswert sich in Abhängigkeit der Relativbewegung dieser beiden Bauteile ändert. Umfasst sind jedoch auch Gestaltungen, bei denen der Sensor zwischen der Basis und einem Zwischenbauteil angeordnet ist, wobei das Zwischenbauteil mit der Betätigungshandhabe mechanisch zwangsgekoppelt ist.

Was den Typ des erfindungsgemäßen Spenders angeht, so sind verschiedene Ausgestaltungen denkbar. So kann die Betätigungshandhabe eines erfindungsgemäßen Spenders mechanisch mit einer Pumpeinrichtung gekoppelt sein, die eine volumetrisch veränderliche Pumpkammer aufweist, deren Volumen durch Betätigen der Betätigungshandhabe verkleinert werden kann. Bei einem solchen Spender wird somit eine Charge des auszutragenden Mediums zum Zwecke des Austrags druckbeaufschlagt und entweicht dann aus dem Spender. Üblicherweise und vorzugsweise weist ein solcher Spender ein druckabhängig öffnendes Auslassventil jenseits der Pumpkammer auf, welches durch Druckbeaufschlagung seitens der Flüssigkeit in der Pumpkammer geöffnet wird. Bei einem Spender mit einer Pumpeinrichtung und insbesondere einem solchen mit einem druckabhängig öffnenden Auslassventil besteht eine große Zahl an Einflussfaktoren, die dazu führen kann, dass typgleiche Spender dennoch in verschiedenen Stellungen der Betätigungshandhabe den Austragvorgang beginnen. Die erfindungsgemäße Gestaltung mit einem Kalibrierungsspeicher mit darin abgelegtem spenderspezifischem Verhalten ist daher gerade bei dieser Art von Spendern von Vorteil.

Ein erfindungsgemäßer Spender kann jedoch auch derart ausgebildet sein, dass die Betätigungshandhabe ohne Zwischenschaltung eines druckbeaufschlagten Fluids mechanisch mit einem Auslassventil gekoppelt ist, das durch Verlagerung der Betätigungshandhabe geöffnet werden kann. Bei einem solchen Spender wirkt die Betätigungshandhabe auf das Auslassventil somit nicht mittelbar über den Druck der Flüssigkeit in der Pumpkammer, sondern unmittelbar durch mechanische Kopplung. Dennoch gibt es auch bei diesem Typ Spender eine Vielzahl von Einflussfaktoren, die dazu führen können, dass unterschiedliche Relativstellungen der Betätigungshandhabe gegenüber dem Basiskörper bei typgleichen Spendern den Austragvorgang beginnen lassen.

Die Spender des letztgenannten Typs können insbesondere auch Spender sein, in deren Flüssigkeitsspeicher das auszutragende Medium unter Druck gelagert wird. Das Auslassventil kann insbesondere mit einer Dosierkammer gekoppelt sein, die eine stets im Wesentlichen gleichbleibende Menge des Mediums von jenem im Flüssigkeitsspeicher verbleibenden Medium isoliert und im Zuge der Betätigung des Auslassventils ausgibt.

Die Erfindung betrifft darüber hinaus auch ein Verfahren zur Herstellung eines Spenders eingangs bezeichneter Art. Dabei wird mittels einer nicht dem Spender zugehörigen Kalibrierungseinrichtung automatisiert eine Betätigung des Spenders durchgeführt und dabei das Verhalten des Spenders erfasst. Nachfolgend werden Daten im Kalibrierungsspeicher des Spenders abgelegt, die das erfasste Verhalten des Spenders repräsentieren.

Vorzugsweise weist ein erfindungsgemäßer Spender zu diesem Zwecke eine von außen zugängliche elektronische Schnittstelle auf, mittels derer ein Kalibrierungsvorgang ausgelöst werden kann. Wenn über diese Schnittstelle ein Signal an die CPU des Spenders gesendet wird, schreibt diese den aktuell am Sensor erfassten Sensorwert in den Kalibrierungsspeicher des Spenders.

Bei genannten Verfahren ist vorgesehen, dass noch im Zuge des Herstellungsprozesses des Spenders ein Kalibrierungsvorgang durchgeführt wird, mittels dessen das Verhalten eines konkreten Spenders erfasst wird. Hierbei kann es sich insbesondere um eine Erfassung des Beginns eines Austragvorgangs in Abhängigkeit der Relativstellung der Betätigungshandhabe gegenüber dem Basiskörper handeln. Denkbar ist jedoch auch eine alternative oder zusätzliche Erfassung der Stellung der Betätigungshandhabe bei Abschluss des Austragvorgangs. Dieser für den Spender individuell ermittelte Wert wird dann in den Kalibrierungsspeicher geschrieben, so dass er anschließend zur Berücksichtigung bei der Erfassung von Austragvorgängen mit diesem Spender zur Verfügung steht.

Ein erfindungsgemäßer Spender ist zur Durchführung eines Verfahrens zum Betrieb ausgebildet, welches durch folgende Verfahrensschritte in Reaktion auf eine Betätigung des Spenders gekennzeichnet ist. Es wird das Maß der Verlagerung des Referenzbauteils gegenüber dem Basiskörper mit dem genannten Sensor erfasst. Anschließend erfolgt eine Kategorisierung der erfolgten Betätigung durch Vergleich dieses Maßes mit Daten aus dem Kalibrierungsspeicher. In Abhängigkeit der erfolgten Kategorisierung wird dann ein Verarbeitungsschritt in Form eines Zählvorgangs durchgeführt oder nicht durchgeführt. Das Verfahren wird insbesondere in kurzen Zeitabständen während ein und derselben Betätigung wiederholt durchgeführt, beispielsweise im Abstand von 50 Millisekunden.

### KURZBESCHREIBUNG DER ZEICHNUNGEN

Weitere Aspekte und Vorteile der Erfindung ergeben sich aus den Ansprüchen sowie der nachfolgenden Beschreibung eines bevorzugten Ausführungsbeispiels der Erfindung, welches anhand der Figuren erläutert wird. Dabei zeigen:
- Fig. 1: eine erste Ausführungsform eines erfindungsgemäßen Spenders,
- Fig. 2: eine vergrößerte Darstellung des Austragkopfes des Spenders der Fig. 1,
- Fig. 3: eine zweite Ausführungsform eines erfindungsgemäßen Spenders,
- Fig. 4: zwei typgleiche, jedoch verhaltensunterschiedliche, erfindungsgemäße Spender und
- Fig. 5: das Verfahren zur Kalibrierung eines erfindungsgemäßen Spenders.

### DETAILLIERTE BESCHREIBUNG DER AUSFÜHRUNGSBEISPIELE

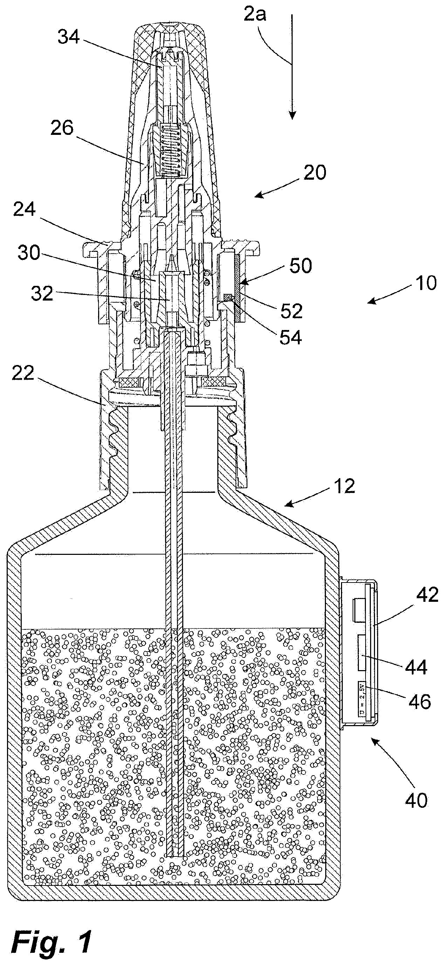

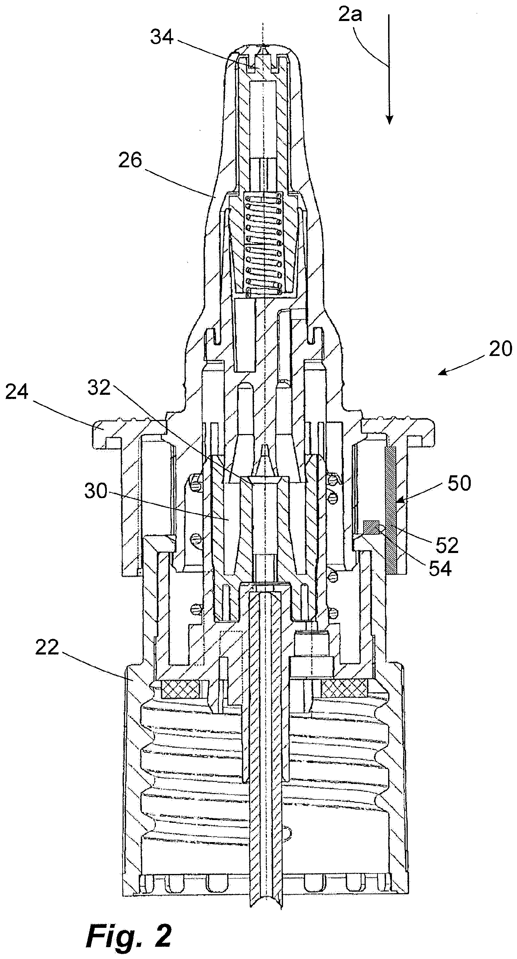

Die Fig. 1 und 2 zeigen eine erste Ausführungsform eines erfindungsgemäßen Spenders sowie dessen Austragkopf in vergrößerter Darstellung.

Der Spender 10 unterscheidet sich hinsichtlich seiner flüssigkeitsführenden Teile nicht von herkömmlichen Spendern. Seine konkrete Ausgestaltung ist exemplarisch zu verstehen.

Der Spender 10 verfügt über einen Flüssigkeitsspeicher 12 und einen Austragkopf 20. Dieser Austragkopf 20 weist einen auf den Flüssigkeitsspeicher 12 aufgeschraubten Basiskörper 22 sowie eine dem gegenüber verlagerbare Betätigungshandhabe 24 auf, die auch eine Nasenolive 26 mit Austragöffnung umfasst.

Der Flüssigkeitsspeicher 12 ist zur Lagerung der auszutragenden pharmazeutischen Flüssigkeit vor dem Austrag vorgesehen. Zum Zwecke des Austrags weist der Austragkopf 20 eine Pumpkammer 30 auf, die einlassseitig mit einem Schieberventil 32 mit dem Flüssigkeitsspeicher 12 verbunden ist und die auslassseitig ein druckabhängig öffnendes Ventil 34 aufweist.

Zur Betätigung dieses Spenders wird die Betätigungshandhabe 24 in Richtung des Pfeils 2a hinabgedrückt, wodurch ein Schließen des Einlassventils 32 verursacht wird. Sobald das Einlassventil 32 geschlossen ist, führt eine fortgesetzte Verlagerung der Betätigungshandhabe 24 nach unten zu einem Druckanstieg der in der Pumpkammer 30 befindlichen Flüssigkeit, welcher seinerseits ein Öffnen des Auslassventils 34 und darauffolgend einen Austragvorgang verursacht.

Der Spender der Fig. 1 unterscheidet sich von rein mechanischen Spendern durch das Vorhandensein einer elektronischen Zählvorrichtung 40, welche bestimmungsgemäß in Reaktion auf einen erfolgten Austragvorgang ein Zählregister erhöht und das Ergebnis auf einer Anzeigevorrichtung 42 anzeigt.

Die Erfassung des Austragvorgangs mit der Zählvorrichtung 40 soll nur erfolgen, wenn der Austragvorgang tatsächlich begonnen wurde, nicht aber, wenn die Verlagerung der Betätigungshandhabe 24 gegenüber dem Basiskörper 22 für einen Austragvorgang nicht ausgereicht hat.

Um die Zählvorrichtung 40 in die Lage zu versetzen, zu erkennen, ob die Verlagerung der Betätigungshandhabe zur Erzeugung eines Austragvorgangs ausgereicht hat, ist ein Sensor 50 vorgesehen, der die Relativbewegung der Betätigungshandhabe 24 gegenüber dem Basiskörper 22 erfasst. Der Sensor 50 ist als Potentiometer ausgebildet und verfügt über eine Widerstandsschicht 52 sowie einen Gleitkontakt 54. Das Potentiometer wirkt als Spannungsteiler. Zwischen den beiden gegenüberliegenden Enden der Widerstandsschicht 52 liegt eine Spannung von beispielsweise 5 V an. Die zwischen dem unteren Ende und der Widerstandsschicht 52 und dem Gleitkontakt 54 abfallende Teilspannung hiervon steigt mit zunehmender Verlagerung der Betätigungshandhabe 24 in Richtung des Pfeils 2a nach unten an.

Das Potentiometer ist in nicht näher dargestellter Weise mit einer CPU 44 der Zähleinrichtung 40 verbunden, welche in der Lage ist, die durch die Relativlage des Gleitkontaktes 54 gegenüber der Widerstandsschicht 52 sich ergebende Teilspannung zwischen dem Gleitkontakt 54 und dem unteren Ende der Widerstandsschicht 52 zu erfassen. Da der Gleitkontakt 54 auf Seite des Basiskörpers 22 angebracht ist und die Widerstandsschicht 52 auf Seiten der Betätigungshandhabe 24, wird im Zuge der Betätigungsbewegung in Richtung des Pfeils 2a die genannte Teilspannung kontinuierlich verändert. Die CPU 44 wertet die Teilspannung aus und vergleicht den Wert mit einem im Speicher 46 gespeicherten Spannungswert, vorliegend rein beispielhaft einem Wert von 2,5 V. Mit ansteigender Verlagerung der Betätigungshandhabe 24 gegenüber dem Basiskörper 22 steigt diese Teilspannung. Wenn der Grenzwert von 2,5 V, der im Speicher 46 hinterlegt ist, überschritten wird, so wird der genannte Zählschritt durch die CPU 44 durchgeführt und das Ergebnis auf der Anzeigevorrichtung 42 dargestellt.

Der jeweilige Grenzwert, der im Speicher 46 hinterlegt ist, hängt von dem jeweils individuellen Spender ab. Bei dem Speicher handelt es sich daher vorzugsweise um einen einmalig oder mehrfach beschreibbaren Speicher. Er ist in im Weiteren noch beschriebener Weise im Zuge des Herstellungsprozesses ermittelt und im Speicher hinterlegt worden.

Durch den spenderspezifisch vorgehaltenen Wert wird die Gefahr verringert, dass ein tatsächlich erfolgter Austragvorgang fälschlicherweise nicht gezählt wird oder eine Verlagerung der Betätigungshandhabe 24 gegenüber dem Basiskörper 22 trotz Nichteintretens des Austragvorgangs gezählt wird.

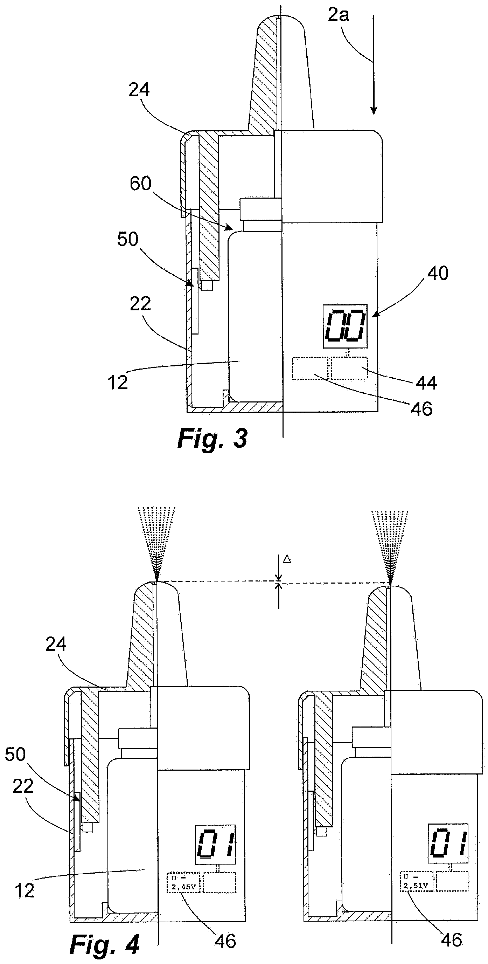

Die Austragvorrichtung der Fig. 3 ist hinsichtlich des Vorhandenseins einer Zähleinrichtung 40 sowie eines als Potentiometer ausgestalteten Sensors 50 mit der Ausgestaltungsform der Fig. 1 und 2 weitgehend identisch. Allerdings ist die Bauart dieses Spenders eine grundsätzlich andere, da er keine Pumpkammer aufweist. Im Gehäuse, welches durch einen Basiskörper 22 und eine Betätigungshandhabe 24 gebildet ist, ist ein Pumpspender 60 angeordnet, der seinerseits über einen Flüssigkeitsspeicher 12 verfügt. In diesem ist die auszutragende Flüssigkeit unter Druck gelagert. Ein Austragvorgang kann bewirkt werden, indem ein Auslassstutzen 25 gemeinsam mit der Betätigungshandhabe 24 niedergedrückt wird. Dies führt zu einem Öffnen eines Auslassventils unter Abgabe einer vorher in einer internen Dosierkammer abgemessenen Flüssigkeitsmenge.

Auch bei dieser Ausgestaltung wertet eine CPU 44 unter Berücksichtigung eines spenderindividuell in einem Speicher 46 gespeicherten Spannungswertet aus, ob die Betätigungshandhabe 24 ausreichend weit verlagert wurde, so dass voraussichtlich ein Austragvorgang stattgefunden hat.

Die Fig. 4 verdeutlicht, dass typgleiche Spender sich hinsichtlich der Relativlage der Betätigungshandhabe 24 gegenüber dem Basiskörper 22 unterscheiden können. Die linke und die rechte Teildarstellung der Fig. 4 zeigt solche typgleichen Spender jeweils in jener Relativstellung der Betätigungshandhabe 24 gegenüber dem Basiskörper 22, in der das Auslassventil des Pumpspenders 60 öffnet. Die gekennzeichnete Wegdifferenz Δ in den jeweils erforderlichen Verlagerungswegen der Betätigungshandhabe 24 wirkt sich auf die Teilspannung am Sensor 50 zwischen einem Ende der Widerstandsschicht 52 und dem Gleitkontakt 54 im Augenblick des beginnenden Austragvorgangs aus. Dementsprechend sind in den Speicher 44 auch unterschiedliche Spannungswerte eingetragen, die durch die CPU 44 mit dem Wert des Sensors 50 verglichen werden, um zu erfassen, ob ein Austragvorgang stattgefunden hat.

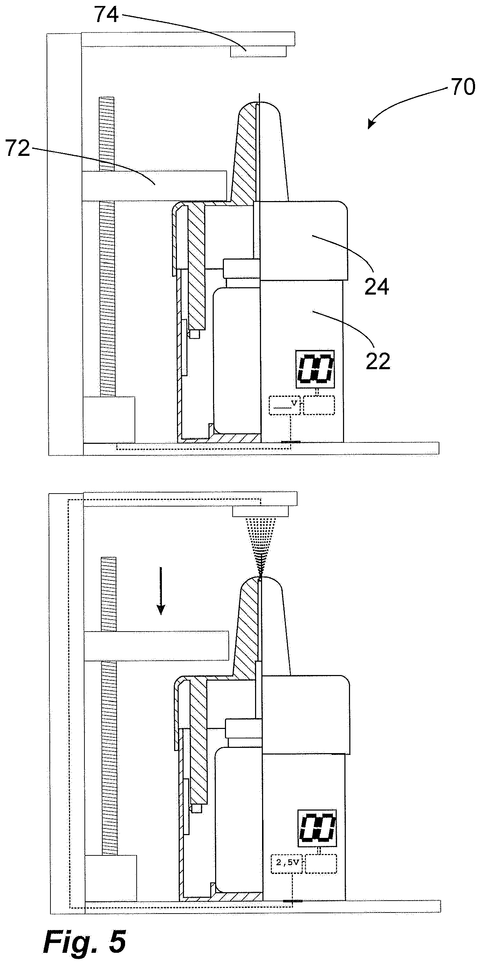

Die Fig. 5 zeigen in schematisierter Art und Weise das Verfahren zur Kalibrierung des Spenders der Fig. 3 und 4. Dieser ist in eine Kalibrierungseinrichtung 70 eingesetzt, die dafür ausgebildet ist, mittels eines verfahrbaren Drückers 72 die Betätigungshandhabe 24 gegenüber dem Basiskörper 22 niederzudrücken. Oberhalb der Austragöffnung des Spenders ist ein Sensor 74 vorgesehen, der das Eintreten des beginnenden Austragvorgangs erfassen kann. Er kann beispielsweise als Feuchtigkeits- oder Drucksensor ausgebildet sein. Während die Austragvorrichtung 10 in die Kalibrierungsvorrichtung 70 eingesetzt ist, ist eine Datenverbindung zwischen der CPU 44 und dem genannten Sensor 74 gegeben. Hierfür können an der Unterseite des Spenders elektrische Kontakte vorgesehen sein.

Nach Einsetzen des Spenders wird der Betätigungsdrücker 72 nach unten verlagert. Sobald der Sensor 74 im Zustand der Fig. 5 den erfolgenden Austragvorgang registriert und dies an die CPU 44 kommuniziert, schreibt diese den in jenem Zeitpunkt vom Sensor 50 erfassten Spannungswert in den Speicher 46. Der Spender ist dann kalibriert.

## Patentansprüche

1. Spender (10) zum Austrag pharmazeutischer Medien mit
- einem Flüssigkeitsspeicher (12),
- einem Basiskörper (22),
- einer gegenüber dem Basiskörper (22) manuell verlagerbaren Betätigungshandhabe (24) zur Verursachung eines Austragvorgangs von Medium aus dem Flüssigkeitsspeicher (12) in eine Umgebung und
- einem elektronischen Erfassungssystem zur Erfassung von Austragvorgängen,
wobei das Erfassungssystem aufweist:
- einen Sensor (50), der in Abhängigkeit der Verlagerung eines Referenzbauteils, welches mit der Betätigungshandgabe (24) mechanisch gekoppelt ist, so dass es im Zuge eines Austragvorgangs gegenüber dem Basiskörper (22) verlagert wird, ein Ausgangssignal erzeugt, wobei der Sensor (50) zur Erzeugung von Ausgangssignalen mindestens zwei unterscheidbarer Arten in Abhängigkeit des Maßes der Verlagerung ausgebildet ist, und
- einen elektronischen Kalibrierungsspeicher (46), in welchem Kalibrierungsdaten zum Austragverhalten des Spenders (10) abgelegt sind, welche individuellen Besonderheiten des Spenders (10) in Hinblick auf sein Verhalten in Reaktion auf eine Verlagerung der Betätigungshandhabe (24) repräsentieren,
wobei
- das Erfassungssystem eine Auswerteeinrichtung (40) zur Verarbeitung der Austragssignale aufweist, welche den Austragvorgang unter Berücksichtigung der Art des Ausgangssignalen auswertet,
- die Auswerteeinrichtung (40) als Zähleinrichtung ausgebildet ist, welche den Austragvorgang unter Berücksichtigung der Art des Ausgangssignals und unter Berücksichtigung von im Kalibrierungsspeicher (46) abgelegten Daten auswertet, und
- wobei der Spender zur Durchführung eines Betriebsverfahrens ausgebildet ist, bei dem bei Betätigung des Spenders (10)
a. das Maß der Verlagerung des Referenzbauteils gegenüber dem Basiskörper (22) durch den Sensor (50) erfasst wird,
b. eine Kategorisierung der Betätigung durch Vergleich des Maßes der Verlagerung mit Daten aus dem Kalibrierungsspeicher (46) erfolgt, und
c. in Abhängigkeit der Kategorisierung ein Zählvorgang durchgeführt beziehungsweise nicht durchgeführt wird.

2. Spender (10) nach Anspruch 1,
**dadurch gekennzeichnet, dass**
der Sensor (50) zur Erzeugung eines analogen Ausgangssignals ausgebildet ist, wobei der Sensor (50) insbesondere als Potentiometer (50) ausgebildet ist.

3. Spender (10) nach einem der vorstehenden Ansprüche,
**dadurch gekennzeichnet, dass**
die Betätigungshandhabe (24) mechanisch mit einer Pumpeinrichtung gekoppelt ist, die eine volumetrisch veränderliche Pumpkammer (30) aufweist, deren Volumen durch die Betätigungshandhabe (24) verkleinert werden kann.

4. Spender nach einem der Ansprüche 1 bis 2,
**dadurch gekennzeichnet, dass**
die Betätigungshandhabe (24) mechanisch mit einem Auslassventil gekoppelt ist, das durch Verlagerung der Betätigungshandhabe (24) geöffnet werden kann.

5. Verfahren zur Herstellung eines Spenders (10) nach einem der vorstehenden Ansprüche,
**dadurch gekennzeichnet, dass**
a. mittels einer nicht dem Spender zugehörigen Kalibrierungseinrichtung (70) automatisiert eine Betätigung des Spenders erfolgt, wobei das Verhalten des Spenders erfasst wird, und
b. Daten im Kalibrierungsspeicher (46) des Spenders (10) abgelegt werden, die das erfasste Verhalten des Spenders repräsentieren.

6. Verfahren nach Anspruch 5,
**dadurch gekennzeichnet, dass**
bei der Erfassung der Verhaltens des Spenders (10) erfasst wird, in welcher Relativstellung des Referenzbauteils (24) zum Basiskörper (22) während der Betätigung,
- der Austrag des Mediums beginnt und/oder
- der Austrag des Mediums abgeschlossen ist.

## Claims

1. Dispenser (10) for discharging pharmaceutical media, with
- a liquid reservoir (12),
- a base body (22),
- an actuating handle (24) which is manually displaceable with respect to the base body (22) in order to cause a process involving discharge of medium from the liquid reservoir (12) into an environment, and
- an electronic detection system for detecting discharge processes,
wherein
the detection system has:
- a sensor (50) which generates an output signal depending on the displacement of a reference component that is mechanically coupled to the actuating handle (24) such that it is displaced with respect to the base body (22) during a discharge process, wherein the sensor (50) is configured to generate output signals of at least two distinguishable types as a function of the extent of the displacement, and
- an electronic calibration memory (46), in which calibration data concerning the discharge behavior of the dispenser (10) are stored, which data represent individual peculiarities of the dispenser (10) in respect of its behaviour in reaction to a displacement of the actuating handle (24),
wherein
- the detection system has an evaluation device (40) for processing the output signals, which evaluation device (40) evaluates the discharge process by taking account of the type of the output signals,
- the evaluation device (40) is configured as a counting device which evaluates the discharge process by taking account of the type of the output signal and taking account of data stored in the calibration memory (46), and
- wherein the dispenser is configured to carry out an operating method in which, upon actuation of the dispenser (10),
a. the extent of the displacement of the reference component with respect to the base body (22) is detected by the sensor (50),
b. the actuation is categorized by comparing the extent of the displacement with data from the calibration memory (46), and
c. a counting process is performed or not performed, depending on the categorization.

2. Dispenser (10) according to Claim 1,
**characterized in that**
the sensor (50) is configured to generate an analog output signal, wherein the sensor (50) is configured in particular as a potentiometer (50).

3. Dispenser (10) according to either of the preceding claims,
**characterized in that**
the actuating handle (24) is mechanically coupled to a pump device which has a volumetrically variable pump chamber (30), of which the volume can be decreased by the actuating handle (24).

4. Dispenser according to either of Claims 1 and 2,
**characterized in that**
the actuating handle (24) is mechanically coupled to an outlet valve which can be opened by displacement of the actuating handle (24).

5. Method for producing a dispenser (10) according to one of the preceding claims,
**characterized in that**
a. an actuation of the dispenser takes place in an automated manner by means of a calibration device (70) not assigned to the dispenser, wherein the behavior of the dispenser is detected, and
b. data representing the detected behavior of the dispenser are stored in the calibration memory (46) of the dispenser (10).

6. Method according to Claim 5,
**characterized in that**
the detection of the behavior of the dispenser (10) involves detecting the relative position of the reference component (24) to the base body (22) in which, during the actuation,
- the discharge of the medium begins and/or
- the discharge of the medium is concluded.

## Revendications

1. Distributeur (10) destiné à distribuer des fluides pharmaceutiques, comprenant
- un réservoir à liquide (12),
- un corps de base (22),
- une poignée d'actionnement (24) dont la position peut être changée manuellement par rapport au corps de base (22) et destinée à causer une opération de distribution de fluide depuis le réservoir à liquide (12) dans un environnement et
- un système de détection électronique destiné à détecter les opérations de distribution,
le système de détection possédant :
- un capteur (50) qui génère un signal de sortie en fonction du changement de position d'un élément structural de référence qui est couplé mécaniquement à la poignée d'actionnement (24) de telle sorte qu'il change de position par rapport au corps de base (22) dans le cadre d'une opération de distribution, le capteur (50) étant configuré pour générer des signaux de sortie d'au moins deux types différents en fonction de l'amplitude du changement de position, et
- une mémoire d'étalonnage électronique (46) dans laquelle sont stockées des données d'étalonnage à propos du comportement de distribution du distributeur (10), lesquelles représentent des particularités individuelles du distributeur (10) du point de vue de son comportement en réaction à un changement de position de la poignée d'actionnement (24),
dans lequel
- le système de détection possède un dispositif d'interprétation (40) destiné à traiter les signaux de sortie, lequel interprète l'opération de distribution en tenant compte du type des signaux de sortie,
- le dispositif d'interprétation (40) est réalisé sous la forme d'un dispositif de comptage qui interprète l'opération de distribution en tenant compte du type du signal de sortie et en tenant compte des données stockées dans la mémoire d'étalonnage (46), et
- le distributeur étant configuré pour mettre en oeuvre un procédé opérationnel lors duquel, lors de l'actionnement du distributeur (10)
a. l'amplitude du changement de position de l'élément structural de référence par rapport au corps de base (22) est détectée par le capteur (50),
b. une catégorisation de l'actionnement est effectuée par comparaison de l'amplitude du changement de position avec des données issues de la mémoire d'étalonnage (46), et
c. une opération de comptage est exécutée ou non exécutée, en fonction de la catégorisation.

2. Distributeur (10) selon la revendication 1, **caractérisé en ce que** le capteur (50) est configuré pour générer un signal de sortie analogique, le capteur (50) étant notamment réalisé sous la forme d'un potentiomètre (50).

3. Distributeur (10) selon l'une des revendications précédentes, **caractérisé en ce que** la poignée d'actionnement (24) est couplée mécaniquement avec un dispositif de pompage, lequel possède une chambre de pompe (30) à volumétrie variable dont le volume peut être réduit par la poignée d'actionnement (24).

4. Distributeur selon l'une des revendications 1 à 2, **caractérisé en ce que** la poignée d'actionnement (24) est couplée mécaniquement à une vanne de sortie qui peut être ouverte par le changement de position de la poignée d'actionnement (24).

5. Procédé de fabrication d'un distributeur (10) selon l'une des revendications précédentes,
**caractérisé en ce que**
a. un actionnement du distributeur est effectué de manière automatisée au moyen d'un dispositif d'étalonnage (70) qui ne fait pas partie du distributeur, le comportement du distributeur étant détecté, et
b. des données sont stockées dans la mémoire d'étalonnage (46) du distributeur (10), lesquelles représentent le comportement détecté du distributeur.

6. Procédé selon la revendication 5,
**caractérisé en ce que**
la détection du comportement du distributeur (10) inclut la détection de la position relative de l'élément structural de référence (24) par rapport au corps de base (22) pendant l'actionnement dans laquelle
- commence la distribution du fluide et/ou
- la distribution du fluide est terminée.
